(19) Europäisches Patentamt
European Patent Office
Office européen des brevets

(11) EP 0 627 225 B1

(12) **EUROPÄISCHE PATENTSCHRIFT**

(45) Veröffentlichungstag und Bekanntmachung des Hinweises auf die Patenterteilung:
**13.09.2000 Patentblatt 2000/37**

(51) Int. Cl.[7]: **A61L 15/00**, B01J 20/24

(21) Anmeldenummer: **94105114.6**

(22) Anmeldetag: **31.03.1994**

(54) **Superabsorbentien und ein Verfahren zu ihrer Herstellung**

Superabsorbents and process of their production

Superabsorbants et procédé de fabrication

(84) Benannte Vertragsstaaten:
**AT BE CH DE DK ES FR GB GR IE IT LI LU MC NL PT SE**

(30) Priorität: **01.06.1993 DE 4318094**

(43) Veröffentlichungstag der Anmeldung:
**07.12.1994 Patentblatt 1994/49**

(73) Patentinhaber:
**Chemische Fabrik Stockhausen GmbH**
**47805 Krefeld (DE)**

(72) Erfinder: **Kelkenberg, Heike, Dr.**
**D-45966 Gladbeck (DE)**

(74) Vertreter:
**Wolff, Felix, Dr. et al**
**Kutzenberger & Wolff**
**Theodor-Heuss-Ring 23**
**50668 Köln (DE)**

(56) Entgegenhaltungen:
**EP-A- 0 212 688** **EP-A- 0 424 672**
**WO-A-90/10426**

- **DATABASE WPI Week 8717, Derwent Publications Ltd., London, GB; AN 87-120043 & JP-A-62 064 803 (NICHIDEN KAGAKU)**

EP 0 627 225 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft neue Superabsorbentien, ein Verfahren zu ihrer Herstellung sowie ihre Anwendung im Hygienebereich.

Superabsorbentien, stark wasserabsorbierende und wasserquellbare Polymere, finden vielfältige Verwendung im Sanitär- und Hygienesektor, in Tapetenkleistern, als Trocknungsmittel, als Feuchthaltemittel in der Landwirtschaft oder als Elektrolytverdicker in Trockenbatterien.

Bekannte Absorptionsmittel für diese Verwendungszwecke bestehen aus synthetischen Polymeren oder aus Stärke- oder Cellulose-Pfropfpolymeren, deren Hydrophilie auf einem hohen Anteil an Carboxylgruppen beruht. Bei den vollsynthetischen Polymeren handelt es sich zumeist um schwach vernetzte Polymere, wie teilvernetzte Polyacrylsäuresalze oder teilvernetzte Polymaleinsäurederivate. Sie zeigen die vergleichsweise beste Saugwirkung, werden jedoch biologisch nicht abgebaut.

Geeignete Pfropfpolymerisate sind derivatisierte, meist mit wasserlöslichen Vinylmonomeren gepfropfte Polysaccharide, wie Carboxylmethylcellulose, hydrolysierte Stärke-Acrylnitril-Pfropfpolymere oder Acrylsäure-Stärke-Pfropfpolymere. Sie zeigen im Vergleich zu den vollsynthetischen Polymeren ein deutlich geringeres Absorptionsvermögen für Wasser oder wäßrige Flüssigkeiten. Vorteilhaft ist jedoch der anteilmäßige biologische Abbau dieser natürlichen Quellmittel. Die Herstellung solcher Pfropfpolymeren ist jedoch sehr aufwendig, und die Menge des Biopolymeren im Endprodukt ist durch die hohe Viskosität des Reaktionsmediums, wie dies z. B. bei einer Monomerlösung mit gelöster Stärke der Fall ist, stark begrenzt. Diesbezüglich sei hier nur auf EP-A-0 168 390 und EP-A-0 188 489 verwiesen.

Aus DE-A-35 05 920 ist bekannt, daß schwach vernetzte Polymerisate oder Mischpolymerisate, deren Hydrophilie auf einem hohen Anteil an kationischen Gruppen beruht, gegenüber den bekannten anionischen Absorptionsmitteln zu bevorzugen sind. Insbesondere gegenüber Salzlösungen sind diese weniger empfindlich und zeigen ein deutlich besseres Absorptionsvermögen.

Die WO 90/10426 beschreibt absorbierende Trockenmischungen aus Carbonsäuren mit einem wesentlichen hydrophoben Anteil, verzweigten komplexen Kohlenhydraten und optionalen Vernetzern, wobei die Mischungen beim Zutritt von hydratisierenden Flüssigkeiten vernetzen können. Die hydrophoben Anteile der Carbonsäuren bilden zusatzliche, lamellare Vernetzungsstrukturen aus. Als bevorzugte Carbonsäuren mit wesentlichen hydrophoben Anteilen werden Fettsäuren wie etwa Laurin- oder Palmitinsäure genannt, von den verzweigten Kohlenhydraten wird Carboxymethylcellulose bevorzugt und als Vernetzer kommen vor allem Metallorganische Verbindungen in Frage. Chitosan wird als Komponente erwähnt, aber nicht in den Beispielen verarbeitet. Lange Gelierungszeiten bei Flüssigkeitszutritt und ungebundene Vernetzerbestandteile machen die Mischungen für einen Einsatz in Hygieneprodukten ungeeignet.

EP 424 672 A1 beschreibt vernetzte Chitosane, die zunächst in Wasser als Salz einer organischen Säure gelöst werden, dann in einem organischen Lösemittel als granuläre Partikel ausgefällt und durch eine Alkaliwaschung von der Carbonsäure wieder befreit werden, d.h. in die salzfreie Form überführt werden. Anschließend erfolgt die Vernetzung des Chitosans. Aufgrund der durch den Fällungsprozesses entstehenden besonderen granularen Struktur, weisen die vernetzten Chitosane adsorbierende Eigenschaften für Endotoxine auf. Die salzfreie Struktur dieser vernetzten Chitosanen läßt keine absorbierenden Eigenschaften erwarten, flüssigkeitsbsorbierende, quellende Eigenschaften werden auch nicht beschrieben.

Geeignete kationische Absorptionsmittel sind u. a. Mischpolymerisate auf Basis von radikalisch polymerisierbaren quaternären Ammoniumverbindungen und Acrylamid. Diese vollsynthetischen kationischen Polymerisate sind wie die vollsynthetischen anionischen Polymerisate biologisch nicht abbaubar.

Aufgabe der vorliegenden Erfindung war es, biologisch abbaubare kationische Absorptionsmittel auf Basis natürlicher Biopolymerer bereitzustellen.

Die Aufgabe wird überraschend durch Superabsorbentien gemäß Anspruch 1 gelöst. Die Produkte, Chitosan-Salze, weisen eine hohe Sauggeschwindigkeit und Absorptionskapazität auf.

Die Produkte enthalten pro Gramm Chitosan vorzugsweise 1 bis 30, im besonderen 2 bis 10 mmol Säure.

Vorzugsweise sind die Chitosan-Salze mit Hilfe eines Vernetzungsmittels vernetzt. Sie enthalten dann im allgemeinen 0,0001 bis 10 mmol Vernetzungsmittel pro Gramm Chitosan.

Ein weiterer Gegenstand der Erfindung ist ein Verfahren zur Herstellung der Superabsorbentien auf Chitosan-Basis. Danach erhält man die Chitosan-Salze, indem man pulverförmiges Chitosan in einem organischen Lösemittel mit einer Säure umsetzt. Bei der Reaktion werden vorzugsweise die obengenannten Säuremengen angewandt.

Die Produkte können während der Reaktion mit der Säure oder danach mit einem Vernetzungsmittel vernetzt werden. Dabei wendet man bevorzugt 0,0001 bis 10 mmol Vernetzungsmittel pro Gramm Chitosan an.

Nach Mahlung ist das Chitosan pulverförmig bei Korngrößen $\leq$ 1 mm. Das eingesetzte Pulver besitzt im allgemeinen jedoch weniger als 10 Gewichtsprozent Teilchen mit Korngrößen < 1 $\mu$m.

Chitosan stellt chemisch ein aminogruppenhaltiges Polysaccharid mit einer der Cellulose analogen linearen Struktur dar.

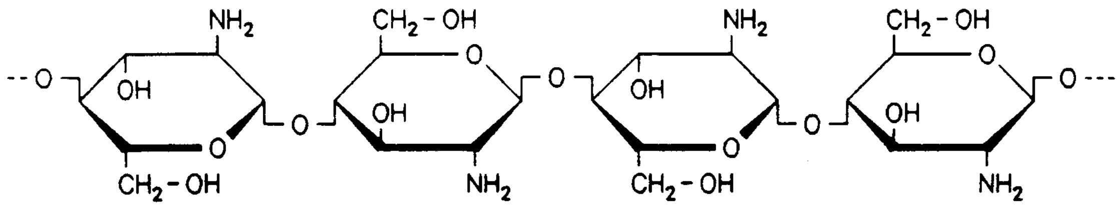

Struktur des Chitosans (Ausschnitt)

Chitosan hat in den letzten Jahren als nachwachsender Rohstoff hauptsächlich in Japan und den USA an wirtschaftlicher Bedeutung gewonnen. Es wird durch Deacetylierung von Chitin, einem Abfallprodukt aus der Krabbenfischerei produziert. Chitin ist nach der Cellulose das zweithäufigste Polysaccharid der Erde.
Das marktgängige Chitosan enthält noch ca. 20 % acetylierte Aminogruppen, hat ein Molgewicht von 300 000 bis 500 000 und ist in den meisten organischen Lösemitteln und in Wasser unlöslich. Dagegen ist das Chitosan in verdünnten Säuren löslich. Aufgrund der hohen Molmasse des Chitosans sind diese Lösungen sehr hochviskos, so daß Reaktionen nur in sehr hoher Verdünnung durchgeführt werden können. Das Aufarbeiten solcher Lösungen stellt technisch ein großes Problem dar.

Es war daher überraschend, daß sich Chitosan in heterogener Phase als Pulver in einem organischen Lösemittel suspendiert mit Säuren zur Reaktion bringen läßt. Der Habitus des Pulvers bleibt dabei erhalten. Das Produkt kann als Feststoff gehandhabt werden.

Als organisches Lösemittel sind Alkohole, wie beispielsweise Methanol, Ethanol, Propanol oder Isopropanol, Butanol, Pentanol, Cyclohexanol u. a., geeignet. Daneben lassen sich aber auch Ketone, wie beispielsweise Aceton, Methylethylketon, Methylisobutylketon oder Diisobutylketon, verwenden. Auch Ester, wie beispielsweise Methylacetat oder Ethylacetat, können eingesetzt werden. Am besten eignen sich Lösemittel, in denen die zur Reaktion zu bringende Säure löslich ist. Außerdem sind solche Lösemittel zu bevorzugen, die geringe Mengen Nasser zu lösen vermögen.

Bevorzugte Lösemittel sind Methanol, Ethanol, Aceton oder Butanol. Es können auch Lösemittelgemische angewandt werden.

Das marktgängige Chitosan sollte möglichst in gemahlenem Zustand mit einer Korngröße von ≤ 500 µm eingesetzt werden. Doch auch das technisch unbehandelte Chitosan ist entsprechend reaktiv. Das Verhältnis Chitosan zu Lösemittel ist variabel und richtet sich nach dem verwendeten Reaktionsgefäß. Die Suspension sollte jedoch nicht zu verdünnt sein, um einen möglichst vollständigen Umsatz der Säure zu gewährleisten.

Das Gewichtsverhältnis von Lösemittel zu Chitosan liegt meist im Bereich von 1 : 1 bis 100 : 1, vorzugsweise bei 5 : 1 bis 20 : 1. Bei einem Verhältnis von 1 : 1 läßt sich das Gemisch bereits im Kneter bzw. in einem Extruder mit einer Säure zur Reaktion bringen. In einem üblichen Rührreaktor arbeitet man bevorzugt mit einem Mischungsverhältnis von 10 : 1.

Die Reaktion wird vorzugsweise in Gegenwart geringer Gehalt an Wasser vorgenommen. Die Wassermenge beträgt dann meist 1 bis 200 %, vorzugsweise 1 bis 80 %, bezogen auf das eingesetzte Chitosan. Das Wasser quillt das in der organischen Phase suspendierte Chitosan etwas an.

Die Umsetzung mit der Säure wird meist im Temperaturbereich von 15 bis 150 °C vorgenommen. Temperaturen von 40 bis 90 °C werden dabei bevorzugt. Während bei Zimmertemperatur mitunter bis zu 30 Stunden benötigt werden, läßt sich die Reaktion im bevorzugten Temperaturbereich oder bei der Siedetemperatur des Lösemittels im allgemeinen in 1 bis 8 Stunden durchführen.

Sowohl anorganische als auch organische Säuren lassen sich in der Suspension mit Chitosan zur Reaktion bringen. Dabei werden starke Säuren vorzugsweise eingesetzt. Geeignet sind beispielsweise Mineralsäuren, Sulfonsäuren, Carbonsäuren mit 1 bis 4 Carboxylgruppen oder Hydroxycarbonsäuren. Man kann auch Säuremischungen anwenden. Abhängig von der Säure werden Reaktionsprodukte mit sehr unterschiedlichen Eigenschaften erhalten. Mit Salzsäure wird beispielsweise ein Produkt erhalten, das als Pulver in Wasser kalt löslich ist und als Lösung eine erstaunlich geringe Viskosität aufweist.

Die Schwefelsäure- und Phosphorsäure-Produkte stellen dagegen wasserunlösliche Produkte dar, die sich möglicherweise über eine Salzvernetzung erklären lassen.

Ameisensäure liefert ein Reaktionsprodukt, das trübe in Wasser löslich ist und eine hohe Viskosität zeigt.

Ein überraschendes Ergebnis zeigen die Hydroxysäuren, wie beispielsweise Milchsäure und Glykolsäure.

Mit ihnen bilden die Chitosan-Salze in Wasser glasklare Gele.

Vorzugsweise werden für die Superabsorbentien organische Säuren und dabei im besonderen Hydroxysäuren verwendet.

Die Quellbarkeit der Chitosan-Salze läßt sich durch Nachvernetzung weiter verbessern. Als Vernetzungsmittel kommen alle polyfunktionellen Substanzen infrage, die mit Amino- oder OH-Gruppen zu reagieren vermögen, wobei die Zahl der funktionellen Gruppen bevorzugt zwei sein sollte.

Beispiele für Vernetzungsmittel sind im Sinne der Erfindung Dicarbonsäuren, Dianhydride, Dicarbonsäurechloride, Diepoxide oder Dialdehyde. Die Vernetzungsmittel werden vorzugsweise so ausgewählt, daß sich die gebildeten Vernetzungsstellen biologisch oder hydrolytisch leicht wieder öffnen lassen, um den gewünschten Bioabbau sicherzustellen.

Da es sich bei den Chitosan-Salzen um sehr lange Polymerketten handelt, ist nur eine sehr geringe Menge an Vernetzerkomponente erforderlich, um eine optimale Vernetzung zu erreichen. Bei zu starker Vernetzung wird die Quellfähigkeit negativ beeinflußt und geht deutlich zurück. Die Menge an zugesetztem Vernetzungsmittel ist abhängig von seiner Art. Bei den Dicarbonsäuren, Dianhydriden und den Säurechloriden muß eine vergleichsweise große Menge an Vernetzungsmittel von 0,01 bis 10 mmol/g Chitosan eingesetzt werden, da diese Vernetzungsmittel teilweise mit dem vorzugsweise in kleinen Mengen vorliegenden Nasser oder mit den Alkoholen, wenn diese als Lösemittel verwendet waren, abreagieren können. Diepoxide und Dialdehyde dagegen reagieren bevorzugt mit den Aminogruppen des Chitosan-Salzes, so daß diese beispielsweise nur in Mengen von $10^{-4}$ bis $10^{-2}$ mmol/g Chitosan angewandt werden.

Im Falle der Dialdehyde als Vernetzungsmittel werden mit den Aminogruppen des Chitosan-Salzes als Vernetzerstellen Aldimin-Bindungen ausgebildet, die im Abwasser oder sonst in der Umwelt leicht wieder hydrolytisch durch Wasser aufgespalten werden und deshalb biologisch gut abgebaut werden. Die Epoxid-vernetzten Produkte zeigen im Vergleich zu den Dialdehyd-vernetzten Produkten einen leicht verzögerten biologischen Abbau.

Durch die Nachvernetzung wird beispielsweise das Wasserabsorptionsvermögen der Chitosan-Milchsäure und Chitosan-Glyoxylsäure deutlich erhöht.

Die erfindungsgemäßen biologisch abbaubaren Produkte zeigen insbesondere gegenüber synthetischem Urin ein gutes Aufnahmevermögen. Sie eignen sich deshalb in besonderem Maße zur Einarbeitung in umweltfreundliche zellstoffhaltige, saugfähige Hygieneartikel, wie Wegwerfwindeln, Damenbinden, Wischtüchern und Krankenunterlagen. Sie können außerdem in Tapetenkleistern, als Trocknungsmittel sowie als Wasserspeicher oder Feuchthaltemittel in der Landwirtschaft und in kosmetischen und pharmazeutischen Produkten verwendet werden.

Die Erfindung soll durch folgende Beispiele verdeutlicht werden.

Zur Charakterisierung des Flüssigkeitsabsorptionsvermögens sowie der biologischen Abbaubarkeit werden folgende Bestimmungsmethoden herangezogen:

<u>Saugwert</u>

Ca. 0,5 g Produkt werden auf eine Glasfritte (Typ G3, Durchmesser 50 mm) gestreut, die mit einer mit vollentsalztem Wasser oder synthetischen Urin gefüllten Bürette verbunden und auf das Niveau der Glasfritte nivelliert ist. Die absorbierte Flüssigkeitsmenge wird nach 0,5 und 10 Minuten an der Bürette gemessen.

$$\text{Saugwert} = \frac{\text{absorbierte Flüssigkeitsmenge}}{\text{Einwaage}} \ (\text{ml/g})$$

Der Anfangswert nach 0,5 Minuten ist ein Maß für die Sauggeschwindigkeit. Der Endwert nach 10 Minuten ist ein Maß für die Absorptionskapazität.

Zusammensetzung des synthetischen Urins:

| | |
|---|---|
| 970,77 g | VE-Wasser |
| 8,77 g | Natriumchlorid |
| 0,93 g | Magnesiumsulfat mit 6 Kristallwasser |
| 19,40 g | Harnstoff |

<u>Biologischer Abbau</u>

Der biologische Abbau wird im sogenannten modifizierten Sturm-Test nach OECD-Norm (301 B adopted:

12.05.81) untersucht.

In ein 5-l-Gefäß werden 2,5 l Nährlösung gefüllt und mit 30 ml Klärschlamm (Überstand) eine kommunalen Kläranlage versetzt. Diese Mischung wird 24 Stunden mit $CO_2$-freier Luft begast. Anschließend werden drei Absorptionsgefäße mit jeweils 100 ml 0,025 M NaOH hinter das 5-l-Gefäß in Reihe geschaltet. Nach Zugabe von 60 ml einer wäßrigen 0,1%igen Lösung der zu testenden Substanz wird der Kolben mit 413 ml VE-Wasser auf 3 l aufgefüllt (als Kontrollsubstanz dient Natriumbenzoat).

Nährsalz-Gehalt:

| | |
|---|---|
| 22,50 mg/l | $MgSO_4$ x 7 $H_2O$ |
| 40,00 mg/l | $(NH_4)_2SO_4$ |
| 27,50 mg/l | $CaCl_2$ (wasserfrei) |
| 1,00 mg/l | $FeCl_3$ x 6 $H_2O$ |
| 17,00 mg/l | $KH_2PO_4$; 43,5 mg/l $K_2HPO_4$ |
| 66,80 mg/l | $Na_2HPO_4$ x 7 $H_2O$ |
| 3,40 mg/l | $NH_4Cl$ |

Das Gefäß wird mit 50 bis 100 ml/min $CO_2$-freier Luft begast, wobei das durch den biologischen Abbau entstehende $CO_2$ in den Absorptionsgefäßen als $Na_2CO_3$ zurückgehalten wird. Das verbleibende NaOH wird innerhalb der ersten 10 Tage jeden zweiten Tag, anschließend jeden fünften Tag mit 0,05 M HCl potentiometrisch titriert. Am Schluß der Messung wird 1 ml konzentrierte Salzsäure zur Freisetzung des anorganischen Carbonats zugesetzt. Als Bezugsgröße dient der Gehalt der Testsubstanz an organisch gebundenem Kohlenstoff.

$$CO_2\text{-Produktion} = [\text{ml HCl (Blindwert)} - \text{ml HCl (Test)}] \times 1{,}10\ (\text{mg } CO_2)$$

$$\%\ \text{Bioabbau} = \frac{\text{mg } CO_2 \text{ prod.}}{60\ \text{mg} \times CO_2\text{-Gehalt (theor.)}} \times 100$$

In den Beispielen 1 bis 7 wird das Verfahren zur Herstellung von Chitosan-Salzen näher erläutert.

<u>Beispiel 1</u>

Chitosan-HCl-Salz

103 g methanolische Salzsäure (entspr. 80 mmol HCl) und 10 g Wasser werden in einem Rührkolben kalt vorgelegt und mit 10 g gemahlenem Chitosanpulver (< 500 µm) unter starkem Rühren versetzt. Danach wird bis zum Rückfluß erhitzt. Nach 4 Stunden Reaktionszeit ist die Säureaufnahme beendet. Nach dem Abkühlen wird zentrifugiert, der Rückstand zweimal mit Methanol gewaschen und bis zur Gewichtskonstanz getrocknet.
Auswaage: 11,7 g
Das Pulver ist in kaltem Wasser löslich.
Eine 1%ige Lösung zeigt keine veränderte Viskosität gegenüber Wasser.

<u>Beispiel 2</u>

Chitosan-Ameisensäure-Salz

Analog Beispiel 1 werden 10 g Chitosanpulver mit 80 mmol Ameisensäure umgesetzt.
Auswaage: 10,5 g
Das Pulver ist in kaltem Wasser trübe löslich.
Eine 1%ige Lösung zeigt eine erhöhte Viskosität.

Beispiel 3

Chitosan-Glykolsäure-Salz

Analog Beispiel 1 werden 10 g technisches Chitosan ungemahlen mit 80 mmol Glykolsäure umgesetzt.
Auswaage: 13,6 g
Das Pulver ist in kaltem Wasser klar löslich.
Eine 1%ige Lösung ist hochviskos und kaum noch fließfähig.

Beispiel 4

Chitosan-3-Hydroxybuttersäure-Salz

Analog Beispiel 1 werden 10 g gemahlenes Chitosanpulver mit 80 mmol 3-Hydroxybuttersäure umgesetzt.
Auswaage: 10,7 g
Das Produkt quillt in kaltem Wasser leicht an, ist jedoch unlöslich.

Beispiel 5

Chitosan-Milchsäure-Salz

Analog Beispiel 1 werden 10 g gemahlenes Chitosanpulver mit 80 mmol Milchsäure umgesetzt.
Auswaage: 13,6 g
Das Produkt quillt stark in kaltem Wasser.
Eine 1%ige Lösung bildet ein klares, fast stehendes Gel.

Beispiel 6

Chitosan-Milchsäure-Salz

100 g Ethanol, 7,4 g Wasser und 9,8 g Milchsäure werden vorgelegt und unter Rühren mit 10 g gemahlenem Chitosanpulver versetzt. Danach wird 3 Stunden unter Rückfluß erhitzt. Nach dem Abkühlen wird filtriert und der Filterrückstand mit Ethanol gewaschen und getrocknet.
Auswaage: 13,1 g
Das Produkt quillt stark in kaltem Wasser.
Eine 1%ige Lösung stellt ein klares bewegliches Gel dar.

Beispiel 7

Chitosan-Milchsäure-Salz

Die Herstellung erfolgt analog Beispiel 6 unter Einsatz von Aceton anstelle von Ethanol als Lösemittel.
Auswaage: 13,5 g
Das Produkt quillt stark in kaltem Wasser.
Eine 1%ige Lösung stellt ein klares bewegliches Gel dar.

Beispiele 8 bis 21

Die Beispiele 8 bis 21 beschreiben mit Glutaraldehyd vernetzte Chitosan-Milchsäure-Salze.
Die Vernetzungsreaktion wird in verschiedenen Lösemitteln nach folgender Methode durchgeführt:

> 100 g Lösemittel und 10 g Wasser werden vorgelegt und unter Rührung mit 13 g Chitosan-Milchsäure-Salz (Beispiel 5) versetzt. Nach 10 Minuten Quellzeit werden langsam unter starker Rührung 3 ml einer 5mmolaren Lösung von Glutardialdehyd im Lösemittel (= 0,015 mmol) hinzugetropft. Danach wird 1 Stunde auf 58 °C erhitzt. Nach dem Abkühlen wird filtriert, der Filterkuchen mit wenig Lösemittel gewaschen und bei 50 °C im Vakuum getrocknet.

In der folgenden Tabelle sind die Saugwerte der in verschiedenen Lösemitteln hergestellten vernetzten Produkte zusammengefaßt.

| Mit Glutardialdehyd vernetztes Produkt | | Saugwert in vollentsalztem Wasser (ml/g) | |
|---|---|---|---|
| Bsp. | Lösemittel | 0,5 Minuten | 10 Minuten |
| 8 | Aceton | 42,9 | 83,0 |
| 9 | 1-Butanol | 36,2 | 84,0 |
| 10 | Ethylmethylketon | 15,2 | 21,7 |
| 11 | Isopropanol | 33,7 | 68,6 |
| 12 | Cyclohexanol | 24,4 | 48,8 |
| 13 | 2-Ethylhexan-1-ol | 21,3 | 45,7 |
| 14 | Methanol | 12,5 | 54,2 |
| 15 | Ethylacetat | 14,3 | 19,4 |
| 16 | Methylacetat | 28,1 | 78,1 |

Die Beispiele 17 bis 21 beschreiben vernetzte Chitosan-Milchsäure-Salze, die in Aceton mit unterschiedlichen Mengen Glutardialdehyd als Vernetzungsmittel hergestellt werden.

| Vernetztes Produkt | | Saugwert in vollentsalztem Wasser (ml/g) | |
|---|---|---|---|
| Bsp. | Glutardialdehyd (mmol/g Prod.) | 0,5 Minuten | 10 Minuten |
| 17 | 0,00 | 35,6 | 70,2 |
| 18 | 0,0008 | 42,9 | 80,4 |
| 19 | 0,00115 | 42,9 | 83,0 |
| 20 | 0,0023 | 37,2 | 76,6 |
| 21 | 0,0307 | 28,0 | 68,0 |

<u>Beispiele 22 bis 27</u>

Die Beispiele 22 bis 27 beschreiben mit Sorbitoldiglycidylether vernetzte Chitosan-Milchsäure-Salze.

Die Vernetzungsreaktion wird in verschiedenen Lösemitteln nach folgender Methode durchgeführt:

> 100 g Lösemittel und 10 g Wasser werden vorgelegt und unter Rührung mit 13 g Chitosan-Milchsäure-Salz (Beispiel 5) versetzt. Nach 10 Minuten Quellzeit werden langsam unter starker Rührung 4 ml einer 5mmolaren Lösung von Glutardialdehyd im Lösemittel (= 0,02 mmol) hinzugetropft. Danach wird 1 Stunde auf 58 °C erhitzt. Nach dem Abkühlen wird filtriert, der Filterkuchen mit wenig Lösemittel gewaschen und bei 50 °C im Vakuum getrocknet.

In der folgenden Tabelle sind die Saugwerte der in verschiedenen Lösemitteln hergestellten vernetzten Produkte zusammengefaßt.

| Mit Sorbitoldiglycidylether vernetztes Produkt | | Saugwert in vollentsalztem Wasser (ml/g) | |
|---|---|---|---|
| Bsp. | Lösemittel | 0,5 Minuten | 10 Minuten |
| 22 | Aceton | 38,9 | 87,7 |
| 23 | 1-Butanol | 31,0 | 63,0 |
| 24 | Ethylmethylketon | 23,2 | 35,2 |
| 25 | 2-Ethylhexan-1-ol | 30,2 | 59,4 |
| 26 | Methanol | 33,0 | 61,4 |
| 27 | Methylacetat | 27,6 | 56,9 |

| Saugwerte in synthetischem Urin (ml/g) | | |
|---|---|---|
| Bsp. | 0,5 Minuten | 10 Minuten |
| 9 | 16,6 | 25,4 |
| 19 | 16,2 | 27,5 |
| 22 | 13,0 | 24,9 |

| Biologischer Abbau (%) | | | | |
|---|---|---|---|---|
| Tage | Natriumbenzoat | Bsp. 5 | Bsp. 19 | Bsp. 22 |
| 3 | 30,3 | 3,7 | 3,4 | 3,5 |
| 5 | 52,4 | 30,0 | 21,0 | 19,1 |
| 7 | 58,9 | 44,4 | 32,5 | 29,6 |
| 10 | 65,8 | 53,6 | 42,7 | 37,9 |
| 14 | 71,3 | 60,5 | 50,0 | 52,9 |
| 19 | 73,9 | 64,3 | 53,6 | 55,8 |
| 24 | 76,4 | 70,5 | 57,2 | 58,7 |
| 28 | 78,6 | 72,8 | 60,2 | 60,4 |
| 32 | 79,3 | 75,2 | 62,3 | 61,1 |

## Patentansprüche

1. Superabsorbentien erhältlich durch Reaktion von pulverförmigem Chitosan suspendiert in einer organischen Säure, ausgenommen Carbonsäuren mit einem substantiellen hydrophoben Bereich und Abtrennen des pulverförmigen Reaktionsproduktes aus der Suspension.

2. Superabsorbentien, dadurch gekennzeichnet, daß Chitosan mit einer Hydroxycarbonsäure umgesetzt wird.

3. Superabsorbentien nach Anspruch 2, dadurch gekennzeichnet, daß die Hydroxycarbonsäure Milchsäure, Hydroxybuttersäure und/oder Glykolsäure ist.

4. Superabsorbentien nach einem der Ansprüche 1 bis 3, dadurch gekennzeichnet, daß die Säuremenge 1 bis 30 mmol/g Chitosan beträgt.

5. Superabsorbentien nach Anspruch 2 bis 4, dadurch gekennzeichnet, daß das mit Hydroxycarbonsäure umgesetzte Chitosan mit einem Vernetzungsmittel vernetzt ist.

6. Superabsorbentien nach Anspruch 5, dadurch gekennzeichnet, daß das Vernetzungsmittel Dicarbonsäuren, Dianhydride, Dicarbonsäurechloride, Diepoxide oder Dialdehyde sind.

7. Superabsorbentien nach einem der Ansprüche 5 oder 6, dadurch gekennzeichnet, daß die Menge an Vernetzungsmittel bei 0,0001 bis 10 mmol/g Chitosan liegt.

8. Verfahren zur Herstellung von Superabsorbentien, dadurch gekennzeichnet, daß

- pulverförmiges Chitosan in einem Gemisch aus einem organischen Lösemittel und einer organischen Säure suspendiert wird,
- das suspendierte Chitosan mit der organischen Säure reagiert und
- das pulverförmige Reaktionsprodukt aus der Suspension abgetrennt wird.

9. Verfahren nach Anspruch 8, dadurch gekennzeichnet, daß das Gewichtsverhältnis von Lösemittel zu Chitosan bei 1:1 bis 100:1, vorzugsweise bei 5:1 bis 20:1 liegt.

10. Verfahren nach Anspruch 9, dadurch gekennzeichnet, daß die Säuremenge 1 bis 30, vorzugsweise 2 bis 10 mmol/g Chitosan beträgt.

11. Verfahren nach einem der Ansprüche 8-10, dadurch gekennzeichnet, daß man pulverförmiges Chitosan in einem organischen Lösemittel mit einer organischen Säure umsetzt und gleichzeitig oder danach mit einem Vernetzungsmittel vernetzt.

12. Verfahren nach Anspruch 11, dadurch gekennzeichnet, daß die organische Säure Hydroxycarbonsäure, vorzugsweise Milchsäure, Hydroxybuttersäure und/oder Glykolsäure ist.

13. Verfahren nach Anspruch 11 oder 12, dadurch gekennzeichnet, daß die Menge an Vernetzungsmittel 0,001 bis 10 mmol/g Chitosan beträgt.

14. Verwendung der Superabsorbentien von Anspruch 1-7 in Hygieneartikeln.

**Claims**

1. Superabsorbents which can be obtained by reacting powdered chitosan suspended in an organic acid, with the exception of carboxylic acids having a substantial hydrophobic portion, and removing the powdery reaction product from the suspension.

2. Superabsorbents, characterized in that chitosan is reacted with a hydroxycarboxylic acid.

3. The superabsorbents according to claim 2, characterized in that the hydroxycarboxylic acid is lactic acid, hydroxybutyric acid and/or glycolic acid.

4. The superabsorbents according to any of claims 1 to 3, characterized in that the amount of acid is from 1 to 30 mmol/g chitosan.

5. The superabsorbents according to any of claims 2 to 4, characterized in that the chitosan reacted with a hydroxycarboxylic acid is crosslinked with a cross-linking agent.

6. The superabsorbents according to claim 5, characterized in that the crosslinking agents are dicarboxylic acids,

dianhydrides, dicarboxylic acid chlorides, diepoxides, or dialdehydes.

7. The superabsorbents according to any of claims 5 or 6, characterized in that the amount of crosslinking agent is from 0.0001 to 10 mmol/g chitosan.

8. A process for producing superabsorbents, characterized in that

- powdered chitosan is suspended in a mixture of an organic solvent and an organic acid,
- the suspended chitosan reacts with the organic acid, and
- the powdery reaction product is removed from the suspension.

9. The process according to claim 8, characterized in that the weight ratio of solvent to chitosan is from 1:1 to 100:1, preferably from 5:1 to 20:1.

10. The process according to claim 9, characterized in that the amount of acid is from 1 to 30, preferably from 2 to 10 mmol/g chitosan.

11. The process according to any of claims 8 to 10, characterized in that powdered chitosan in an organic solvent is reacted with an organic acid, and crosslinking with a crosslinking agent is effected simultaneously or subsequently.

12. The process according to claim 11, characterized in that the organic acid is a hydroxycarboxylic acid, preferably lactic acid, hydroxybutyric acid and/or glycolic acid.

13. The process according to claim 11 or 12, characterized in that the amount of crosslinking agent is from 0.001 to 10 mmol/g chitosan.

14. Use of the superabsorbents according to claims 1 to 7 in hygiene articles.

**Revendications**

1. Superabsorbants obtenus par réaction d'un chitosane poudreux mis en suspension dans un acide organique, à l'exception d'acides carboxyliques comportant un domaine hydrophobe substantiel, et séparation du produit de la réaction poudreux de la suspension.

2. Superabsorbants, caractérisés en ce que le chitosane est transformé par réaction avec un acide hydroxycarboxylique.

3. Superabsorbants d'après la revendication 2, caractérisés en ce que l'acide hydroxycarboxylique est de l'acide lactique, de l'acide hydroxybutyrique et/ou de l'acide glycolique.

4. Superabsorbants d'après une des revendications de 1 à 3, caractérisés en ce que la quantité d'acide est de 1 à 3 mmol/g de chitosane.

5. Superabsorbants d'après une des revendications de 2 à 4, caractérisés en ce que le chitosane transformé avec l'acide hydroxycarboxylique est réticulé avec un agent de réticulation.

6. Superabsorbants d'après la revendication 5, caractérisés en ce que l'agent réticulant est un acide dicarboxylique, un dianhydride, un chlorure d'acide dicarboxylique, un diépoxyde ou un dialdéhyde.

7. Superabsorbants d'après une des revendications 5 ou 6, caractérisés en ce que la quantité d'agent réticulant est de 0,0001 à 10 mmol/g de chitosane.

8. Procédé de fabrication de superabsorbants, caractérisé en ce que :

- le chitosane poudreux est mis en suspension dans un mélange composé d'un solvant organique et d'un acide organique
- le chitosane mis en suspension réagit avec l'acide organique et
- le produit de la réaction poudreux est séparé de la suspension.

**9.** Procédé d'après la revendication 8, caractérisé en ce que le ratio en poids du solvant par rapport au chitosane est de 1:1 à 100:1, de préférence de 5:1 à 20:1.

**10.** Procédé d'après la revendication 9, caractérisé en ce que la quantité d'acide est de 1 à 30 mmol/g de chitosane, de préférence de 2 à 10.

**11.** Procédé d'après une des revendications de 8 à 10, caractérisé en ce que l'on transforme le chitosane poudreux dans un solvant organique avec un acide organique et qu'on le réticule simultanément ou ultérieurement avec un agent réticulant.

**12.** Procédé d'après la revendication 11, caractérisé en ce que l'acide organique est de l'acide lactique, de l'acide hydroxybutyrique et/ou de l'acide glycolique.

**13.** Procédé d'après une des revendications 11 ou 12, caractérisé en ce que la quantité d'agent réticulant est de 0,001 à 10 mmol/g de chitosane.

**14.** Utilisation des superabsorbants des revendications 1 à 7 dans les articles d'hygiène.